# EUROPÄISCHE PATENTSCHRIFT

(11) **EP 1 438 286 B1**
(45) Veröffentlichungstag und Bekanntmachung des Hinweises auf die Patenterteilung: **05.05.2010**
(21) Anmeldenummer: 02801325.8
(22) Anmeldetag: 10.10.2002
(51) Int. Cl.: C07C 303/40, C07C 311/65

(54) **VERFAHREN ZUR HERSTELLUNG VON ARYLSULFONSÄUREISOCYANATEN**
METHOD FOR PRODUCING ARYLSUPHONIC ACID ISOCYANATES
PROCEDE DE PRODUCTION D'ISOCYANATES D'ACIDE ARYLSULFONIQUE

(30) Priorität: 11.10.2001 DE 10150368
(43) Veröffentlichungstag der Anmeldung: 21.07.2004
(73) Patentinhaber: BASF SE, 67056 Ludwigshafen (DE)
(72) Erfinder: MAYER, Horst, CEP-12513-300 Guaratingueta (BR); GOLSCH, Dieter, 68165 Mannheim (DE)
(86) Internationale Anmeldenummer: PCT/EP2002/011379
(87) Internationale Veröffentlichungsnummer: WO 2003/033459

(56) Entgegenhaltungen:
- EP-A- 0 021 641
- GB-A- 1 359 428
- DATABASE WPI Section Ch, Week 198335 Derwent Publications Ltd., London, GB; Class E14, AN 1983-751010 XP002227467 ANONYMOUS: "Aryl-sulphonyl isocyanate prepn. - by treating aryl:sulphonamide with phosgene in presence of alkyl isocyanate and aryl-sulphonyl isocyanate" in der Anmeldung erwähnt & RESEARCH DISCLOSURE, Bd. 232, Nr. 010, 10. August 1983 (1983-08-10), Emsworth, GB

## Beschreibung

Die vorliegende Erfindung betrifft ein Verfahren zur Herstellung von Arylsulfonsäureisocyanaten durch Umsetzung eines Arylsulfonamids mit Phosgen in Gegenwart einer katalytisch wirksamen Menge eines Alkylisocyanats.

Arylsulfonylisocyanate sind technisch wichtige Zwischenprodukte bei der Herstellung einer Vielzahl von Verbindungen, insbesondere Herbiziden. Es besteht ein Bedarf an Verfahren zu ihrer Herstellung, die sich neben einer hohen Ausbeute und Produktreinheit auch durch eine hohe Reaktionsgeschwindigkeit und somit kurzen Reaktorbelegzeiten auszeichnen.

Die US 4,379,769 sowie EP-A-0 021 641 beschreibten ein Verfahren zur Herstellung von Arylsulfonylisocyanaten durch Phosgenierung von Arylsulfonamiden in Gegenwart einer katalytisch wirksamen Menge eines Alkylisocyanats und einer katalytisch aktiven Menge einer tertiären Aminbase.

H. Ulrich und A. A. R. Sayigh beschreiben in Angew. Chem. 78, S. 761 - 769 (1966) die Herstellung von Arylsulfonylisocyanaten, wobei man entweder ein Sulfonamid mit einem leicht zugänglichen Alkylisocyanat in das Harnstoffderivat überführt und anschließend phosgeniert, wobei das Ausgangsisocyanat zurückgewonnen wird, oder aber dem Sulfonamid zur Phosgenierung eine katalytische Menge des Isocyanats zusetzt.

In Pestycydy 1989, (4), 1 - 7; ISSN: 0208-8703 wird die Herstellung von 2-Chlorbenzolsulfonsäureisocyanat durch Phosgenierung des entsprechenden Sulfonamids in Gegenwart von Butylisocyanat und in ortho-Dichlorbenzol als Lösungsmittel beschrieben.

In Res. Discl. (1983), 23210, S. 261; ISSN: 0374-4353 wird ein Verfahren zur Herstellung von Arylsulfonylisocyanaten durch Phosgenierung von Arylsulfonamiden beschrieben, bei dem als Katalysator ein Gemisch aus einem Alkylisocyanat und einem Arylsulfonylisocyanat eingesetzt wird. Dabei kann das als Produkt gebildete Arylsulfonylisocyanat in katalytisch wirksamen Mengen in die Reaktion zurückgeführt werden.

Journal of Polymer Science, Vol. 13 (1975), S. 267-268 lehrt zur Synthese von m-Phenylendisulfonyldiisocyanaten durch Phosgenierung von m-Benzoldisulfonamid in Gegenwart katalytischer Mengen eines Alkyl- oder Arylisocyanats eine Mischung aus ortho-Dichlorbenzol und Cellosolv-Acetat als Lösungsmittel einzusetzen.

Der vorliegenden Erfindung liegt die Aufgabe zu Grunde, ein verbessertes Verfahren zur Herstellung von Arylsulfonsäureisocyanaten zur Verfügung zu stellen. Dabei sollen die auftretenden Reaktionszeiten möglichst gering sein und/oder die Bildung unerwünschter Nebenprodukte minimiert werden.

Überraschenderweise wurde nun gefunden, dass diese Aufgabe durch Umsetzung eines Arylsulfonamids mit Phosgen in Gegenwart katalytisch wirksamer Mengen eines Alkylisocyanats gelöst wird, wobei man die Umsetzung zusätzlich in Gegenwart einer katalytisch wirksamen Menge einer Protonensäure oder eines Salzes davon durchführt und/oder wobei die Zudosierung des Phosgens so erfolgt, dass über die Zudosierzeit eine Mindestkonzentration an Alkylarylsulfonylharnstoff im Reaktionsgemisch nicht unterschritten wird.

Gegenstand der vorliegenden Erfindung ist somit ein Verfahren zur Herstellung von Arylsulfonsäureisocyanaten durch Umsetzung eines Arylsulfonamids mit Phosgen, bei dem man in einer Reaktionszone das Arylsulfonamid und eine katalytisch wirksame Menge eines Alkylisocyanats vorlegt, wobei intermediär ein Alkylarylsulfonylharnstoff gebildet wird, und man das Phosgen der Reaktionszone zudosiert, das dadurch gekennzeichnet ist, dass
a) die Umsetzung in Gegenwart einer katalytisch wirksamen Menge einer Protonensäure, die wenigstens eine zur Protolyse befähigte Hydroxygruppe aufweist, oder eines Salzes davon erfolgt, und/oder
b) die Zudosierung des Phosgens so erfolgt, dass über die Zudosierzeit eine Alkylarylsulfonylharnstoffkonzentration von 100 ppm in der Reaktionszone nicht unterschritten wird.

Das erfindungsgemäße Verfahren eignet sich allgemein zur Herstellung von Arylsulfonsäureisocyanaten mit unsubstituierten oder substituierten Arylresten. Dabei handelt es sich beispielsweise um Arylsulfonsäureisocyanate der allgemeinen Formel I 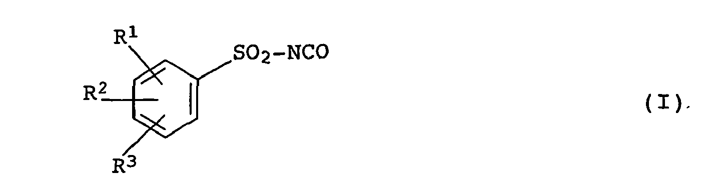 worin
- R¹, R² und R³: unabhängig voneinander für Wasserstoff, jeweils gegebenenfalls substituiertes Alkyl, Cycloalkyl, Heterocycloalkyl, Aryl, Hetaryl sowie WCOOR^{a}, WCOO-M⁺, W(SO₃)R^{a}, W(SO₃)-M⁺, WPO₃(R^{a})(R^{b}), W(PO₃)²-(M⁺)₂, WOR^{a}, WSR^{a}, (CHR^{b}CH₂O)ₓR^{a}, W-Halogen, WNO₂, WC(=O)R^{a} oder WCN stehen,
worin
- W: für eine Einfachbindung, ein Heteroatom oder eine zweiwertige verbrückende Gruppe mit 1 bis 20 Brückenatomen steht,
- R^{a},: E¹, E², E³ jeweils gleiche oder verschiedene Reste, ausgewählt unter Wasserstoff, Alkyl, Cycloalkyl, Heterocycloalkyl, Aryl oder Hetaryl bedeuten,
- R^{b}: für Wasserstoff oder C₁-C₈-Alkyl, bevorzugt Methyl oder Ethyl, steht,
- M⁺: für ein Kationäquivalent steht,
- X⁻: für ein Anionäquivalent steht und
- x: für eine ganze Zahl von 1 bis 20 steht,
wobei jeweils zwei benachbarte Reste R¹, R² und R³ zusammen mit den Kohlenstoffatomen des Benzolrings, an die sie gebunden sind, auch für ein kondensiertes Ringsystem mit 1, 2 oder 3 weiteren Ringen stehen können.

Im Rahmen der vorliegenden Erfindung umfasst der Ausdruck 'Alkyl' geradkettige und verzweigte Alkylgruppen. Vorzugsweise handelt es sich dabei um geradkettige oder verzweigte C₁-C₂₀-Alkyl-, bevorzugterweise C₁-C₁₂-Alkyl- und besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl, Nonyl, Decyl.

Der Ausdruck Alkyl umfasst auch substituierte Alkylgruppen. Substituierte Alkylreste weisen vorzugsweise 1, 2, 3, 4 oder 5, insbesondere 1, 2 oder 3 Substituenten, ausgewählt unter Cycloalkyl, Aryl, Hetaryl, Halogen, NO₂ CN, Acyl, Carboxyl, Carboxylat, -SO₃H und Sulfonat auf.

Der Ausdruck Cycloalkyl umfasst unsubstituierte und substituierte Cycloalkylgruppen. Bei der Cycloalkylgruppe handelt es sich vorzugsweise um eine C₅-C₇-Cycloalkylgruppe, wie Cyclopentyl, Cyclohexyl oder Cycloheptyl.

Wenn die Cycloalkylgruppe substituiert ist, weist sie vorzugsweise 1, 2, 3, 4 oder 5, insbesondere 1, 2 oder 3 Substituenten, ausgewählt unter Alkyl, Alkoxy, Halogen, NO₂, CN, Acyl, Carboxyl, Carboxylat, -SO₃H und Sulfonat auf.

Der Ausdruck Heterocycloalkyl im Sinne der vorliegenden Erfindung umfaßt gesättigte, cycloaliphatische Gruppen mit im allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten, ausgewählt aus Alkyl, Aryl, Alkoxy, Halogen, NO₂, CN, Acyl, COOR^{a}, COO-M⁺ und SO₃R^{a}, bevorzugt Alkyl, tragen können. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Tetrahydrofuranyl, Tetrahydropyranyl, Dioxanyl genannt.

Aryl steht vorzugsweise für Phenyl, Tolyl, Xylyl, Mesityl, Naphthyl, Anthracenyl, Phenanthrenyl, Naphthacenyl und insbesondere für Phenyl oder Naphthyl.

Substituierte Arylreste weisen vorzugsweise 1, 2, 3, 4 oder 5, insbesondere 1, 2 oder 3 Substituenten, ausgewählt unter Alkyl, Alkoxy, Carboxyl, Carboxylat, -SO₃H, Sulfonat, Halogen, NO₂, CN oder Acyl auf.

Hetaryl steht vorzugsweise für Pyrrolyl, Pyrazolyl, Imidazolyl, Indolyl, Carbazolyl, Pyridyl, Chinolinyl, Acridinyl, Pyridazinyl, Pyrimidinyl oder Pyrazinyl.

Substituierte Hetarylreste weisen vorzugsweise 1, 2 oder 3 Substituenten, ausgewählt unter Alkyl, Alkoxy, Carboxyl, Carboxylat, -SO₃H, Sulfonat, Halogen, NO₂, CN oder Acyl auf.

Die obigen Ausführungen zu Alkyl-, Cycloalkyl- und Arylresten gelten entsprechend für Alkoxy-, Cycloalkyloxy- und Aryloxyreste.

Halogen steht für Fluor, Chlor, Brom und Iod, bevorzugt für Fluor, Chlor und Brom.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäure- oder Sulfonsäureesterfunktion oder eine Carbonsäure- oder Sulfonsäureamidfunktion. Dazu zählen z. B. die Ester mit C₁-C₄-Alkanolen, wie Methanol, Ethanol, n-Propanol, Isopropanol, n-Butanol, sec.-Butanol und tert.-Butanol.

M⁺ steht für ein Kationäquivalent, d. h. für ein einwertiges Kation oder den einer positiven Einfachladung entsprechenden Anteil eines mehrwertigen Kations. Vorzugsweise steht M⁺ für ein Alkalimetallkation, wie z. B. Li⁺, Na⁺ oder K⁺ oder für ein Erdalkalimetallkation, für NH₄⁺ oder eine quartäre Ammonium-Verbindung, wie sie durch Protonierung oder Quarternierung von Aminen erhältlich ist. Bevorzugt handelt es sich um Alkalimetallkationen, insbesondere um Natrium- oder Kaliumionen.

X- steht für ein Anionäquivalent, d. h. für ein einwertiges Anion oder den einer negativen Einfachladung entsprechenden Anteil eines mehrwertigen Anions. Vorzugsweise steht X- für ein Carbonat, Carboxylat oder Halogenid, besonders bevorzugt für Cl- und Br-.

Die Werte für x stehen für eine ganze Zahl von 1 bis 240, vorzugsweise für eine ganze Zahl von 3 bis 120.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind ortho-kondensierte Ringsysteme.

Besonders geeignet ist das erfindungsgemäße Verfahren zur Herstellung von Arylsulfonylisocyanaten der Formel 1.1 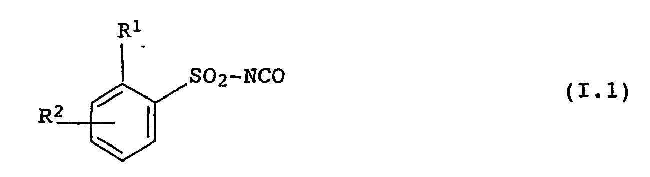 worin
- R¹: für eine elektronenziehende Gruppe, vorzugsweise ausgewählt unter F, Cl, Br, NO₂, CF₂H, CF₂Cl₂, CHCl₂ und CF₃, steht und
- R²: für Wasserstoff, C₁-C₄-Alkyl, C₁-C₄-Alkoxy, F, Cl, Br oder C₁-C₄-Alkylthio steht, wobei die Alkylreste 1, 2 oder 3 Halo-genreste tragen können.

Die als Ausgangsstoffe eingesetzten Sulfonamide lassen sich durch Reaktion der entsprechenden Sulfonsäurechloride mit Ammoniak gewinnen (M. Quaedvlieg in Houben-Weyl, "Methoden der organischen Chemie", Georg Thieme Verlag, Stuttgart, Bd. 9 (1955) 398 - 400, F. Muth, ibid., 605ff).

Die entsprechenden Sulfonsäurechloride zur Herstellung der Sulfonamide erhält man allgemein durch Meerwein-Reaktion (Diazotierung geeigneter Amide und Kupfersalz-katalysierte Sulfochlorierung mit Schwefeldioxid: F. Muth in Houben-Weyl, "Methoden der organischen Chemie", Georg Thieme Verlag, Stuttgart, Bd. 9 (1955) 579, S. Pawlenko in Houben-Weyl, "Methoden der organischen Chemie", Georg Thieme Verlag, Stuttgart, Bd. E 11/2 (1985) 1069), aus den entsprechenden Sulfonsäuren (F. Muth in Houben-Weyl, "Methoden der organischen Chemie", Georg Thieme Verlag, Stuttgart, Bd. 9 (1955) 564), durch Chlorsulfonierung geeigneter aromatischer Vorstufen (F. Muth, ibid., S. 572) oder durch oxidative Chlorierung niederwertiger Schwefelvorstufen (Mercaptane, Diaryldisulfide, S-Benzylmercaptane, Thiocyanate (F. Muth, ibid., S.580, S. Pawlenko, loc. cit., S. 1073).

Vorteilhafterweise kann die Reaktionsgeschwindigkeit bei der Phosgenierung von Arylsulfonamiden gegenüber aus dem Stand der Technik bekannten Verfahren erhöht werden, wenn die Umsetzung in Gegenwart einer katalytisch wirksamen Menge einer Protonensäure, die wenigstens eine zur Protolyse befähigte Hydroxygruppe aufweist, oder eines Salzes davon erfolgt.

Vorzugsweise beträgt die Einsatzmenge der Protonensäure oder des Salzes davon etwa 0,05 bis 1 Gew.-%, besonders bevorzugt 0,1 bis 0,5 Gew.-%, bezogen auf die Menge des eingesetzten Arylsulfonamids.

Geeignete Katalysatoren sind im Allgemeinen Verbindungen des Kohlenstoffs, Stickstoffs, Phosphors und Schwefels, die wenigstens eine zur Protolyse befähigte Hydroxygruppe aufweisen und die Salze davon. Besonders bevorzugt ist der Katalysator ausgewählt unter Carbonsäuren, Salpetersäure, Phosphinsäuren, Phosphonsäuren, Phosphorsäure und deren Mono- und Diestern, Sulfinsäuren, Sulfonsäuren, Schwefelsäure und deren Monoestern und den Salzen davon.

Als Katalysatoren geeignete Salze sind bevorzugt die Alkalimetallsalze, speziell die Li-, Na- und K-Salze.

Bevorzugt wird zur Katalyse der Phosgenierung eine organische Sulfonsäure oder ein Salz davon, insbesondere eine Arylsulfonsäure oder ein Salz davon eingesetzt. Besonders bevorzugt wird Benzolsulfonsäure oder ein Alkalimetallsalz davon, speziell Natriumbenzolsulfonat, eingesetzt.

Alternativ oder zusätzlich zum Einsatz eines Katalysators in Form einer Protonensäure oder eines Salzes davon kann die Reaktionsgeschwindigkeit der Phosgenierung von Arylsulfonamiden gegenüber aus dem Stand der Technik bekannten Phosgenierungsverfahren erhöht werden, wenn die Zudosierung des Phosgens derart erfolgt, dass über die Zudosierzeit eine Alkylarylsulfonylharnstoffkonzentration von 100 ppm, vorzugsweise 500 ppm, in der Reaktionszone nicht unterschritten wird.

Die Bildung des Alkylarylsulfonylharnstoffs in der Reaktionszone erfolgt intermediär aus dem vorgelegten Arylsulfonamid und dem als Katalysator eingesetzten Alkylisocyanat. Mit Zugabe des Phosgens wird das Intermediat zu dem als Produkt angestrebten Arylsulfonsäureisocyanat unter Rückgewinnung des als Katalysator eingesetzten Alkylisocyanats umgesetzt.

Nach einer geeigneten Ausführungsform wird mit der Zudosierung des Phosgens erst begonnen, nachdem die Alkylarylsulfonylharnstoffkonzentration in der Reaktionszone einen Wert von 100 ppm erreicht hat.

Nach einer weiteren geeigneten Ausführungsform wird in der Reaktionszone zusätzlich zu dem Arylsulfonamid und dem Alkylisocyanat der davon abgeleitete Alkylarylsulfonylharnstoff vorgelegt. Die vorgelegte Menge an Alkylarylsulfonylharnstoff beträgt dann mindestens 100 ppm.

Nach einer bevorzugten Ausführungsform wird die Zudosierung des Phosgens über die Zudosierzeit so gesteuert, dass in der Reaktionszone die gewünschte Alkylarylsulfonylharnstoffkonzentration nicht unterschritten wird. Vorzugsweise wird am Anfang der Zudosierzeit ein gegenüber dem maximalen Volumenstrom (Volumenfluss) verringerter Volumenstrom eingesetzt. Bevorzugt wird höchstens während der ersten 40 % der Zudosierzeit, besonders bevorzugt höchstens während der ersten 30 %, insbesondere höchstens während der ersten 20 % ein verringerter Volumenstrom eingesetzt. Der gegenüber dem maximalen Volumenstrom verringerte Strom kann ein Stromprofil aufweisen, das in Form eines Gradienten oder einer oder mehrerer Stufen auf den maximalen Volumenstrom gesteigert wird. Bevorzugt wird am Anfang der Dosierzeit ein gegenüber dem maximalen Volumenstrom konstant verringerter Volumenstrom eingesetzt (Stufenprofil). Bevorzugt beträgt der am Anfang der Dosierzeit eingesetzte Volumenstrom 60 %, besonders bevorzugt 50 % des maximalen Volumenstroms. Besonders bevorzugt ist ein Verfahren, bei dem man während des ersten Sechstels der Zudosierzeit höchstens ein Zehntel der Gesamtphosgenmenge zudosiert.

Vorzugsweise wird am Ende der Zudosierzeit ein gegenüber dem maximalen Volumenstrom verringerter Volumenstrom eingesetzt. Bevorzugt wird höchstens während der letzten 40 % der Zudosierzeit, besonders bevorzugt höchstens während der letzten 30 %, insbesondere höchstens während der letzten 20 % ein gegenüber dem maximalen Volumenstrom verringerter Volumenstrom eingesetzt. Der gegenüber dem maximalen Volumenstrom verringerte Strom kann ein Stromprofil aufweisen, das ausgehend vom maximalen Volumenstrom in Form eines Gradienten oder einer oder mehrerer Stufen verringert wird. Bevorzugt wird am Ende der Dosierzeit ein gegenüber dem maximalen Volumenstrom konstant verringerter Volumenstrom eingesetzt (Stufenprofil). Bevorzugt beträgt der am Ende der Dosierzeit eingesetzte Volumenstrom höchstens 60 %, besonders bevorzugt höchstens 50 % des maximalen Volumenstroms. Besonders bevorzugt ist ein Verfahren, bei dem man während des letzten Sechstels der Zudosierzeit höchstens ein Zehntel der Gesamtphosgenmenge zudosiert. Erfolgt die Zugabe des Phosgens über die gesamte Zudosierzeit mit konstantem Volumenstrom, so muss die Dosierzeit gegenüber der zuvor beschriebenen Rampenfahrweise deutlich verlängert werden. Ansonsten kommt es zu einer merklichen Bildung unerwünschter Nebenprodukte, wie beispielsweise Arylsulfonsäurechloriden, wodurch die Ausbeute an Isocyanaten sinkt.

Nach einer besonders bevorzugten Ausführungsform des erfindungsgemäßen Verfahrens erfolgt die Umsetzung sowohl in Gegenwart einer katalytisch wirksamen Menge einer Protonensäure, wie zuvor beschrieben, oder eines Salzes davon und erfolgt die Steuerung der Zudosierung des Phosgens wie zuvor beschrieben.

Vorzugsweise ist das als Katalysator eingesetzte Alkylisocyanat ausgewählt unter C₄-C₁₀-Alkylisocyanaten und C₅-C₈-Cycloalkylisocyanaten, wie n-Butylisocyanat, n-Pentylisocyanat, n-Hexylisocyanat, n-Octylisocyanat, n-Decylisocyanat und Cyclohexylisocyanat. Bevorzugt wird n-Butylisocyanat eingesetzt. Die Einsatzmenge des Alkylisocyanats liegt vorzugsweise in einem Bereich von 5 bis 40 Mol-%, besonders bevorzugt 10 bis 30 Mol-%, bezogen auf eingesetztes Arylsulfonamid.

Die Einsatzmenge an Phosgen liegt vorzugsweise in einem Bereich von 100 bis 250 Mol-%, besonders bevorzugt 150 bis 200 Mol-%, bezogen auf eingesetztes Arylsulfonamid.

Die Phosgenierung erfolgt vorzugsweise bei einer Temperatur im Bereich von 100 bis 175°C. Der Druck bei der Umsetzung entspricht vorzugsweise Umgebungsdruck, es kann jedoch auch bei erhöhten oder verringerten Drücken gearbeitet werden.

Typische Reaktionszeiten liegen in einem Bereich von etwa 30 Minuten bis 24 Stunden, bevorzugt 1 bis 12 Stunden.

Die Reaktion erfolgt vorzugsweise in einem gegenüber den Edukten inerten Lösungsmittel. Dazu zählen beispielsweise aromatische Kohlenwasserstoffe, wie Toluol, Xylol und Mesitylen, Halogenaromaten, wie Chlorbenzol, halogenierte aliphatische Kohlenwasserstoffe, wie Pentachlorethan etc.

Nach Beendigung der Reaktion kann das Reaktionsgemisch nach üblichen, dem Fachmann bekannten Verfahren aufgearbeitet werden. Dazu zählen beispielsweise Maßnahmen zum Austreiben überschüssigen Phosgens, wie beispielsweise fortgesetztes Erwärmen oder das Durchleiten eines Gasstroms, beispielsweise eines Inertgases, durch die Reaktionslösung. Dazu zählen weiterhin übliche Verfahren zur Abtrennung des eingesetzten Lösungsmittels, wie z. B. Destillation, gegebenenfalls unter vermindertem Druck. Das erfindungsgemäße Verfahren zeichnet sich durch hohe Ausbeuten an Arylsulfonsäureisocyanaten und hohe Produktreinheiten aus. Die nach dem erfindungsgemäßen Verfahren erhaltenen Arylsulfonsäureisocyanate eignen sich in vorteilhafter Weise zur Herstellung von Herbiziden.

Die Erfindung wird anhand der folgenden, nicht einschränkenden Beispiele näher erläutert.

### Beispiel 1

In einem 1 1-Kolben mit Rückflusskühler und Gaseinleitungsrohr werden 112,6 g (0,5 mol) 2-Trifluormethylbenzolsulfonamid; 360 mg Natriumbenzolsulfonat und 9,9 g (0,1 mol) n-Butylisocyanat in 400 g ortho-Xylol vorgelegt und auf eine Innentemperatur von 143 °C erwärmt. Im Verlauf von 2 h werden 12,2 g Phosgen mit im Wesentlichen konstantem Volumenstrom zudosiert. Anschließend werden im Verlauf von weiteren 120 Min. 63,8 g Phosgen bei maximalem Volumenstrom zudosiert. Anschließend werden weitere 11 g Phosgen bei konstantem verringerten Volumenstrom über 2 h zudosiert. Die Ausbeute an 2-Trifluormethylsulfonsäureisocyanat betrug 85 % der Theorie.

### Beispiel 2 (Vergleich)

Es wurde analog zu Beispiel 1 verfahren, wobei jedoch 87 g Phosgen im Verlauf von 7 h mit konstantem Volumenstrom zudosiert wurden. Mittels HPLC wurde die Bildung von ca. 5 % 2-Trifluormethylsulfonsäurechlorid verzeichnet, die Ausbeute an 2-Trifluormethylsulfonsäureisocyanat betrug etwa 80 % der Theorie.

## Patentansprüche

1. Verfahren zur Herstellung von Arylsulfonsäureisocyanaten durch Umsetzung eines Arylsulfonamids mit Phosgen, bei dem man in einer Reaktionszone das Arylsulfonamid und eine katalytisch wirksame Menge eines Alkylisocyanats vorlegt, wobei intermediär ein Alkylarylsulfonylharnstoff gebildet wird, und man das Phosgen der Reaktionszone zudosiert, **dadurch gekennzeichnet, dass**
a) die Umsetzung in Gegenwart einer katalytisch wirksamen Menge einer Protonensäure, die wenigstens eine zur Protolyse befähigte Hydroxygruppe aufweist, oder eines Salzes davon erfolgt, und/oder
b) die Zudosierung des Phosgens so erfolgt, dass über die Zudosierzeit eine Alkylarylsulfonylharnstoffkonzentration von 100 ppm in der Reaktionszone nicht unterschritten wird.

2. Verfahren nach Anspruch 1, bei dem man in Schritt a) eine Protonensäure, die ausgewählt ist unter Carbonsäuren, Salpetersäure, Phosphinsäuren, Phosphonsäuren, Phosphorsäure und deren Mono- und Diestern, Sulfinsäuren, Sulfonsäuren, Schwefelsäure und deren Monoestern, oder ein Salz davon einsetzt.

3. Verfahren nach einem der vorhergehenden Ansprüche, bei dem man in Schritt a) eine organische Sulfonsäure oder ein Alkalimetallsalz davon einsetzt.

4. Verfahren nach einem der vorhergehenden Ansprüche, bei dem während der ersten 40 % der Zudosierzeit ein gegenüber dem maximal eingesetzten Phosgenstrom verringerter Volumenstrom der Reaktionszone zugeführt wird.

5. Verfahren nach einem der vorhergehenden Ansprüche, bei dem während der letzten 40 % der Zudosierzeit ein gegenüber dem maximal eingesetzten Phosgenstrom verringerter Volumenstrom der Reaktionszone zugeführt wird.

6. Verfahren nach einem der vorhergehenden Ansprüche, bei dem man in Schritt b) während des ersten Sechstels der Zudosierzeit höchstens ein Zehntel der Gesamtphosgenmenge zudosiert.

7. Verfahren nach einem der vorhergehenden Ansprüche, bei dem man in Schritt b) während des letzten Sechstels der Zudosierzeit höchstens ein Zehntel der Gesamtphosgenmenge zudosiert.

## Claims

1. A process for preparing arylsulfonyl isocyanates by reacting an arylsulfonamide with phosgene, in which the arylsulfonamide and a catalytically effective amount of an alkyl isocyanate are placed in a reaction zone, forming an alkylarylsulfonylurea as intermediate, and the phosgene is fed into the reaction zone, wherein
a) the reaction is carried out in the presence of a catalytically effective amount of a protic acid which has at least one hydroxy group capable of protolysis or a salt thereof and/or
b) the phosgene is introduced in such a way that the concentration of alkylarylsulfonylurea in the reaction zone does not go below 100 ppm during the time of addition.

2. The process according to claim 1, wherein a protic acid selected from among carboxylic acids, nitric acid, phosphinic acid, phosphonic acids, phosphoric acid and its monoesters and diesters, sulfinic acids, sulfonic acids, sulfuric acid and its monoesters and salts thereof is used in step a).

3. The process according to any of the preceding claims, wherein an organic sulfonic acid or an alkali metal salt thereof is used in step a).

4. The process according to any of the preceding claims, wherein a phosgene stream having a volume flow which is less than the maximum phosgene flow used is fed into the reaction zone during the first 40% of the time of addition.

5. The process according to any of the preceding claims, wherein a phosgene stream having a volume flow which is less than the maximum phosgene flow used is fed into the reaction zone during the last 40% of the time of addition.

6. The process according to any of the preceding claims, wherein, in step b), not more than one tenth of the total amount of phosgene is introduced during the first sixth of the time of addition.

7. The process according to any of the preceding claims, wherein, in step b), not more than one tenth of the total amount of phosgene is introduced during the last sixth of the time of addition.

## Revendications

1. Procédé pour la préparation d'arylsulfonylisocyanates par mise en réaction d'un arylsulfonamide avec du phosgène, dans lequel on dispose au préalable dans une zone de réaction l'arylsulfonamide et une quantité catalytiquement efficace d'un isocyanate d'alkyle, de sorte qu'une alkylarylsulfonylurée est formée intermédiairement, et on introduit par addition dosée le phosgène dans la zone réactionnelle,
**caractérisé en ce que**
a) on effectue la réaction en présence d'une quantité catalytiquement efficace d'un acide protonique qui comporte au moins un groupe hydroxy apte à la protolyse, ou un sel d'un tel acide, et/ou
b) on effectue l'addition dosée du phosgène de manière que pendant le temps de l'addition la concentration d'alkylarylsulfonylurée ne descende pas au-dessous de 100 ppm dans la zone réactionnelle.

2. Procédé selon la revendication 1, dans lequel on utilise dans l'étape a) un acide protonique qui est choisi parmi les acides carboxyliques, l'acide nitrique, les acides phosphiniques, les acides phosphoniques, l'acide phosphorique et ses mono- et diesters, les acides sulfiniques, les acides sulfoniques, l'acide sulfurique et ses monoesters, ou un sel d'un tel acide.

3. Procédé selon l'une quelconque des revendications précédentes, dans lequel on utilise dans l'étape a) un acide sulfonique organique ou un sel de métal alcalin d'un tel acide.

4. Procédé selon l'une quelconque des revendications précédentes, dans lequel pendant les premiers 40 % du temps d'addition dosée on envoie à la zone de réaction un courant volumique réduit par rapport au courant de phosgène introduit au maximum.

5. Procédé selon l'une quelconque des revendications précédentes, dans lequel pendant les derniers 40 % du temps d'addition dosée on envoie à la zone de réaction un courant volumique réduit par rapport au courant de phosgène introduit au maximum.

6. Procédé selon l'une quelconque des revendications précédentes, dans lequel dans l'étape b) pendant le premier sixième du temps d'addition dosée on introduit au maximum un dixième de la quantité totale de phosgène.

7. Procédé selon l'une quelconque des revendications précédentes, dans lequel dans l'étape b) pendant le dernier sixième du temps d'addition dosée on introduit au maximum un dixième de la quantité totale de phosgène.
